# EUROPEAN PATENT SPECIFICATION

(11) **EP 3 830 072 B1**
(45) Date of publication and mention of the grant of the patent: **30.09.2026**
(21) Application number: 19749647.4
(22) Date of filing: 26.07.2019
(51) Int. Cl.: C07C 227/42, A61K 31/196, A61P 29/02, C07C 229/42

(54) **A PROCESS FOR THE PREPARATION OF POLYMORPHIC FORM OF ROBENACOXIB**
VERFAHREN ZUR HERSTELLUNG EINER POLYMORPHEN FORM VON ROBENACOXIB
PROCÉDÉ DE PRÉPARATION D'UNE FORME POLYMORPHE DE ROBENACOXIB

(30) Priority: 27.07.2018 SI 201800166
(43) Date of publication of application: 09.06.2021
(73) Proprietor: KRKA, d.d., Novo mesto, 8000 Novo mesto (SI)
(72) Inventor: ZUPANCIC, David, 1290 Grosuplje (SI); OSTERMAN, Nika, 1430 Hrastnik (SI); PAJK, Matjaz, 8220 Smarjeske Toplice (SI); BERGANT SIMONCIC, Ana, 8210 Trebnje (SI); BENKIC, Primoz, 1000 Ljubljana (SI); SMRKOLJ, Matej, 1420 Trbovlje (SI)
(74) Representative: Kutzenberger Wolff & Partner
(86) International application number: PCT/EP2019/070203
(87) International publication number: WO 2020/021077

(56) References cited:
- EP-A1- 1 007 505
- M. ACEMOGLU, ET AL.: "Synthesis of new N-aryl oxindoles as intermediates for pharmacologically active compounds", TETRAHEDRON, vol. 60, no. 50, 12 October 2004 (2004-10-12), Elsevier Science Publishers, Oxford, GB, pages 11571 - 11586, XP004628620, ISSN: 0040-4020, DOI: 10.1016/J.TET.2004.09.064
- ANON: "Onsior : EPAR - Scientific Discussion", 17 December 2008 (2008-12-17), pages 1 - 27, XP055633693, Retrieved from the Internet <URL:https://www.ema.europa.eu/en/documents/scientific-discussion/onsior-epar-scientific-discussion_en.pdf> [retrieved on 20191018]
- M.R. CAIRA: "Crystalline polymorphism of organic compounds", TOPICS IN CURRENT CHEMISTRY, vol. 198, 1998, Springer Verlag, Berlin, DE, pages 163 - 208, XP001156954, ISSN: 0340-1022, DOI: 10.1007/3-540-69178-2_5

## Description

### Field of the invention

The present invention relates to processes for preparation of polymorphic form D2 of robenacoxib and to pharmaceutical formulations containing it. The polymorphic form of robenacoxib obtained by the process of the present invention is characterized by high polymorphic purity and high chemical and physical stability.

### Background of the invention

Robenacoxib is a common name for {5-ethyl-2-[(2,3,5,6-tetrafluorophenyl) amino]phenyl}acetic acid , a compound of formula (I):

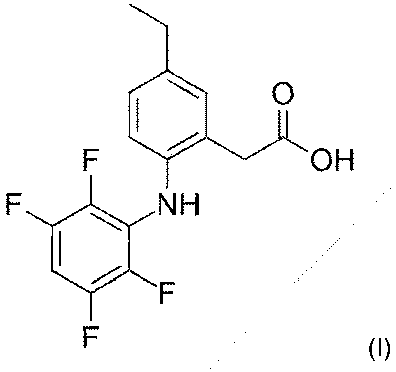

Robenacoxib belongs to the class of COX-2 selective inhibitors and is particularly used for the treatment of rheumatoid arthritis, psoriatic arthritis, osteoarthritis, ankylosing spondylitis, chronic low back pain, acute pain and gout. Like other COX-2 inhibitors, its mechanism of action is by reduction of the generation of prostaglandins (PGs) from arachidonic acid. By blocking the production of prostaglandins, robenacoxib reduces the pain and inflammation caused by musculoskeletal disorders, surgery or osteoarthritis.

Robenacoxib is available in the market under the trademark Onsior, as a tablet in 5 mg, 6 mg, 10 mg, 20 mg, 40 mg strengths and as a solution for injection in concentration of 20 mg of robenacoxib per ml. It is indicated for treatment of acute pain and inflammation associated with musculo-skeletal disorders, with chronic osteoarthritis and with orthopaedic or soft tissue surgery.

According to information obtainable via European Medicines Agency for the product Onsior robenacoxib is a free acid. It is freely soluble in aqueous solution at alkaline pH values above 8. At acidic pH values robenacoxib is practically insoluble in aqueous solution; solubility in pure water is poor. In acid solutions the active substance readily degrades to form a lactam. It is reported that robenacoxib exists in different polymorphic forms and that the more stable form at room temperature was chosen and was shown to be chemically and physically stable under registration stability conditions.

The present inventors have denoted the polymorphic form present in the marketed product as D2.

Robenacoxib polymorphic forms have not been characterized in the literature so far. The basic patent, EP1007505B1, discloses the melting point for robenacoxib crystallized from cyclohexane which is 165-169°C.

In the literature only few methods for robenacoxib preparation are disclosed. EP1007505B1 discloses purification of robenacoxib and analogs thereof by chromatographic purification using ethyl acetate and hexane mixture. In Tetrahedron 2004, Vol:60, Nr:50, pages:11571 - 11586 the precipitation process at pH 3-4 is disclosed in a medium which comprises ethanol, aqueous NaOH and HCl. The inventors of the present invention have found out that the processes disclosed in the prior art result in formation of the polymorphic form which was denoted as D1.

Consequently, there is still a need for an improved, reliable, well defined process which would be reproducible on large scale to prepare polymorphic form D2 in high yields, high purity and in absence of any traces of non-desired forms.

Polymorphic forms are of particular interest due to the provision of essentially pure compounds permitting an exact characterization of physico-chemical properties and possible favorable pharmacological characteristics, such as improved solubility, stability and particle size. There is, however, still a need for a robust process for preparation of polymorphic form essentially free of other polymorphic forms, hydrate forms and amorphous form.

The present inventors have found out that form D2 prepared in essentially pure form resists to any conversion and is stable under technological process conditions such as milling, compression or blending with excipients.

Accordingly, an object of the present invention resides in the provision of a robust, process for the preparation of a polymorphic form of form D2 in essentially pure form in high yields, exhibiting desired pharmacological properties for the preparation of stable pharmaceutical compositions.

### Figures

Figure 1 shows an X-ray diffraction pattern (XRDP) of robenacoxib polymorphic form D1.
Figure 2 shows an XRDP of robenacoxib polymorphic form D2.

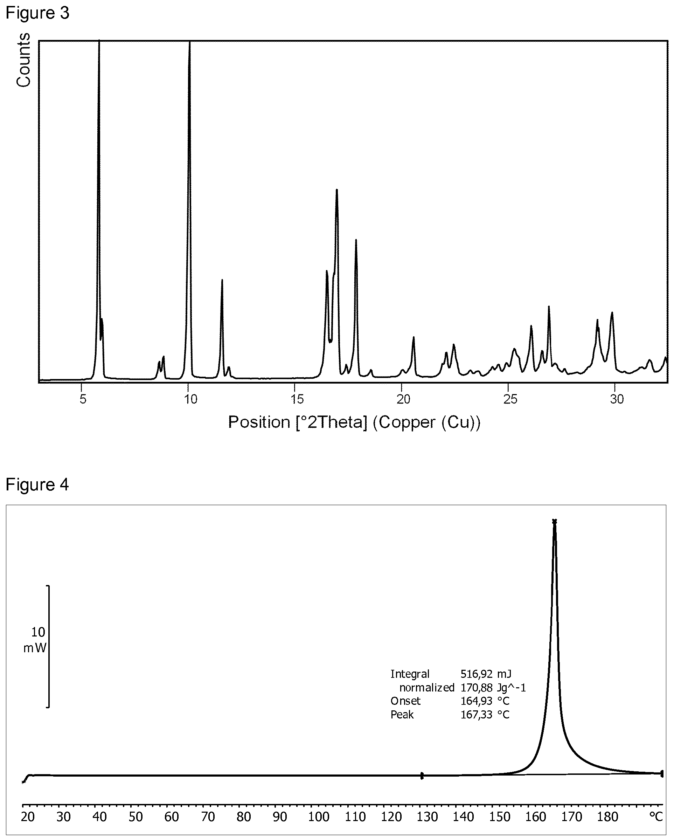

Figure 3 shows an XRDP of robenacoxib mixture of polymorphic forms D1 and D2.
Figure 4 shows a DSC curve of robenacoxib in polymorphic form D1.
Figure 5 shows a scanning electron microscope (SEM) image of robenacoxib in polymorphic form D1.
Figure 6 shows a scanning electron microscope (SEM) image of robenacoxib in polymorphic form D1.
Figure 7 shows a DSC curve of robenacoxib in polymorphic form D2.
Figure 8 shows a scanning electron microscope (SEM) image of robenacoxib in polymorphic form D2.
Figure 9 shows a scanning electron microscope (SEM) image of robenacoxib in polymorphic form D2

### Detailed description of the invention

According to a first embodiment of the present invention a process for the preparation of a stable and crystallographically essentially pure polymorphic form D2 of robenacoxib is provided.

The term "crystallographically essentially pure" means that a polymorphic form comprises less than 30 wt % of other polymorphic forms, amorphous form or solvates, preferably less than 10 wt%, more preferably less than 5 wt %, most preferably less than 2 wt%; even most preferably the form is free of any other polymorphic form, amorphous form or solvate form. Polymorphic purity is determined by means of X-ray powder diffraction (e.g. based on peak intensities).

X-ray powder diffraction data can be obtained, for instance, by a PANalytical X'Pert PRO diffractometer using CuKα radiation of 1.541874 Ⓐ.

Polymorphic form D1 of robenacoxib is characterized by a powder X- ray diffraction pattern with the following peaks (° 2θ ± 0.2°) and inter-planar spacing d [Å]:

| No. | Pos. [°2θ.] | d-spacing [Å] |
|---|---|---|
| 1 | 5.8 | 15.32 |
| 2 | 11.6 | 7.66 |
| 3 | 16.6 | 5.33 |
| 4 | 17.8 | 4.97 |
| 5 | 20.5 | 4.32 |

The polymorphic form D1 is further characterized by a powder X- ray diffraction pattern with the following peaks (° 2θ ± 0.2°) and inter-planar spacing d [Å]:

| No. | Pos. [°2θ.] | d-spacing [Å] |
|---|---|---|
| 1 | 5.8 | 15.32 |
| 2 | 8.8 | 10.04 |
| 3 | 11.6 | 7.66 |
| 4 | 16.6 | 5.33 |
| 5 | 16.8 | 5.27 |
| 6 | 17.8 | 4.97 |
| 7 | 20.5 | 4.32 |
| 8 | 22.5 | 3.95 |
| 9 | 26.9 | 3.32 |
| 10 | 29.1 | 3.06 |

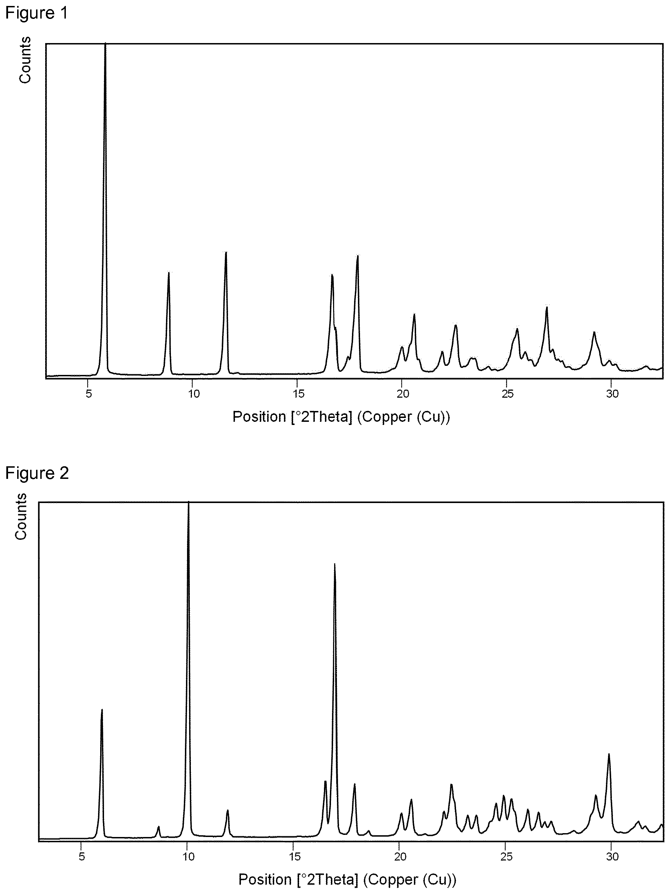

Polymorphic form D1 of robenacoxib is characterized by an XRPD spectrum as substantially shown in Figure 1.

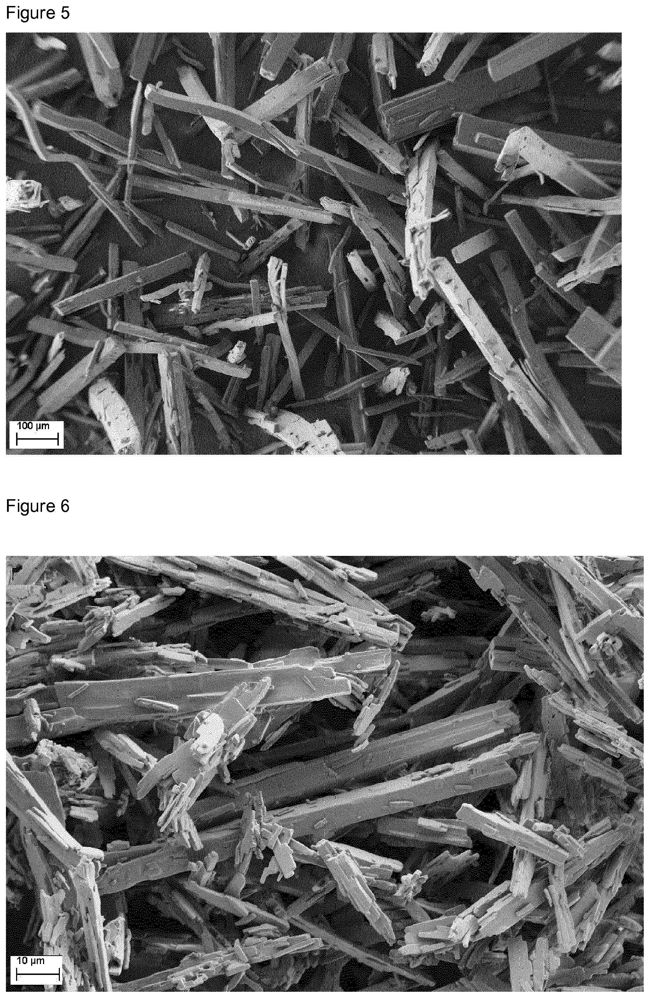

The differential scanning calorimetry (DSC) thermogram of the polymorphic form D1 shows an endothermic peak (melting) at onset temperature 165 °C and Tₚₑₐₖ at about 167 °C. Polymorphic form D1 is further characterized by a DSC thermogram depicted in Figure 5.

Polymorphic form D1 is characterized as an anhydrous and non-hygroscopic crystalline material with a water content of less than about 1% by weight, more preferably less than 0.5% by weight, and most preferably less than 0.2% by weight.

Polymorphic form D1 is further characterized by rod-shaped particle morphology where other morphologies are possible. Adherence of smaller particles onto larger ones and formation of agglomerates is also possible. Scanning electron microscope (SEM) images of robenacoxib in polymorphic form D1 are disclosed in Figure 5 and Figure 6.

Polymorphic form D2 of robenacoxib is characterized by a powder X- ray diffraction pattern with the following peaks (° 2θ ± 0.2°) and inter-planar spacing d [Å]:

| No. | Pos. [°2θ.] | d-spacing [Å] |
|---|---|---|
| 1 | 6.0 | 14.81 |
| 2 | 10.1 | 8.78 |
| 3 | 17.0 | 5.22 |
| 4 | 22.5 | 3.96 |
| 5 | 29.1 | 3.06 |

The polymorphic form D2 may optionally be further characterized by a powder X- ray diffraction pattern with the following peaks (° 2θ ± 0.2°) and inter-planar spacing d [Å]:

| No. | Pos. [°2θ.] | d-spacing [Å] |
|---|---|---|
| 1 | 6.0 | 14.81 |
| 2 | 10.1 | 8.78 |
| 3 | 16.5 | 5.36 |
| 4 | 17.0 | 5.22 |
| 5 | 17.9 | 4.95 |
| 6 | 20.6 | 4.32 |
| 7 | 22.5 | 3.96 |
| 8 | 24.9 | 3.57 |
| 9 | 29.9 | 2.99 |
| 10 | 29.1 | 3.06 |

Polymorphic form D2 of robenacoxib may optionally be further characterized by an XRPD spectrum as shown in Figure 2.

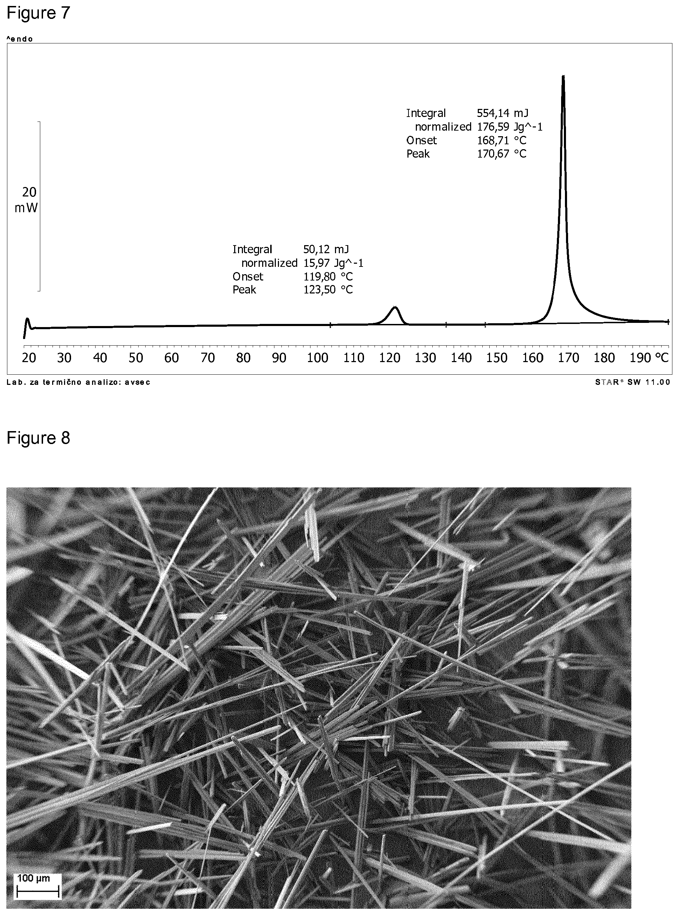

The differential scanning calorimetry (DSC) thermogram of the polymorphic form D2 typically shows an endothermic peak with an onset temperature of about 120°C, which represents a solid-solid phase transition of polymorphic form D2 to form D1 and an endothermic peak (melting of form D1) at onset temperature 169 °C and Tₚₑₐₖ at about 171 °C. Polymorphic form D2 may optionally be further characterized by DSC thermogram depicted on Figure 7.

Hot stage microscopy was performed to provide a confirmation of solid state transitions of robenacoxib form D2. Solid-solid phase transition of form D2 to form D1 was observed in a range from 110 to 130 °C, which is consistent with the DSC thermogram of form D2. Subsequently, transition melting of the form D1 particles obtained from the aforementioned solid-solid phase transition of form D2 to form D1 was detected at about 170 °C.

Polymorphic form D2 is typically characterized as an anhydrous and non-hygroscopic crystalline material with a water content of less than about 1% by weight, more preferably less than 0.5% by weight, and most preferably less than 0,2% by weight.

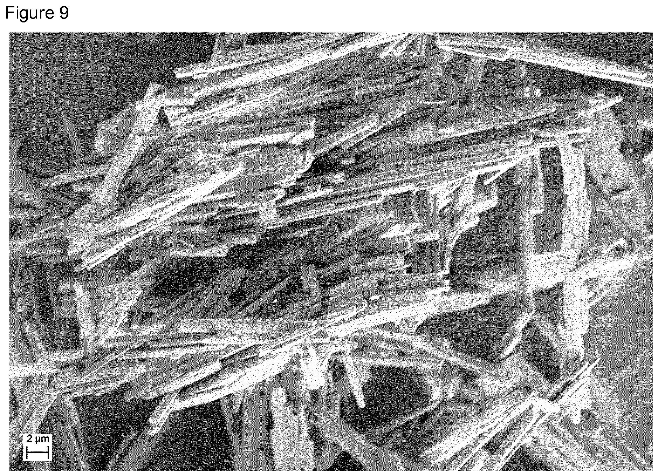

Polymorphic form D2 is typically further characterized by particles of needle-like morphology where other morphologies are also possible. Individual particles of polymorphic form D2 can aggregate to form agglomerates. Scanning electron microscope (SEM) images of robenacoxib in polymorphic form D2 are disclosed in Figure 8 and Figure 9

The process for the preparation of crystallographically essentially pure polymorphic form D2 of robenacoxib according to the present invention comprises:
(i) dissolving robenacoxib in a suitable solvent system A at a temperature in the range of from 0°C to refluxing temperature of said organic solvent system;
(ii) optionally filtering the obtained crystallization mixture;
(iii) optionally cooling the crystallization mixture to a temperature in the range of from 0°C to 50°C or from 40°C to -10°C such as 38°C to -9°C, 36°C to -7°C, 33°C to -6°C or 30°C to -5°C, preferably 30°C to 0°C, more preferably in the range from 10°C to 5°C,
(iv) then
   a. addition of an anti-solvent B to the crystallization mixture to initiate precipitation at crystallization temperature in the range from 50°C to 0°C or from 40°C to -10°C such as 38°C to -9°C, 36°C to -7°C, 33°C to -6°C or 30°C to -5°C, preferably 30°C to 0°C, more preferably in the range from 10°C to 5°C or
   b. addition of the crystallization mixture to an anti-solvent B, to initiate precipitation at crystallization temperature in the range of 50°C to 0°C or 40°C to -10°C such as 38°C to -9°C, 36°C to -7°C, 33°C to -6°C or 30°C to -5°C, preferably 30°C to 0°C, more preferably in the range from 10°C to 5°C
(v) optionally keeping the suspension for a holding time at the crystallization temperature and/or cooling the obtained suspension, wherein the suspension is preferably cooled to a temperature lower than the crystallization temperature and the suspension is kept at said temperature for a holding time
(vi) separating and drying the crystallized polymorphic form D2 of robenacoxib.

The inventors have found out that the crystallization temperature of crystallization process should be carefully controlled in order to obtain a reproducible process resulting in a crystallographically essentially pure polymorphic form D2 of robenacoxib.

The term "crystallization temperature" as used herein relates to the temperature of the crystallization mixture at which the nucleation is induced i.e. the temperature at the onset of the crystallization. In case of the precipitation process the crystallization temperature can be controlled by controlling the temperature of the crystallization mixture. In one embodiment, it is also possible to change the crystallization temperature within the temperature ranges indicated above, i.e. to initiate crystallization at a first temperature selected within at least one of the above temperature ranges and then to hold the obtained suspension at a second temperature within at least one of the above temperature ranges. It is also possible to carry out two or more or even continuous temperature changes during the crystallization provided that all of the selected temperatures during crystallization and holding are within at least one of the above temperature ranges.

In one embodiment, the crystallization batch is kept at crystallization temperature for a holding time for crystal formation for at least 10 minutes, preferably in the range of 10 minutes to 20 hours and more preferred 0.5 to 10 hours. After said holding time at the crystallization temperature, the crystalline particles of robenacoxib are optionally cooled to a temperature lower than crystallization temperature or isolated from the mother liquor using conventional separation techniques, e.g. filtration or centrifugation.

According to the invention, the solvent system A is selected from ketones, such as acetone, methyl ethyl ketone, methyl isobutyl ketone, and mixtures thereof. The preferred solvent system A comprises acetone. Most preferably, the solvent system A consists of acetone.

According to the invention, the anti-solvent B is selected from water, halogenated hydrocarbons such as dichloromethane, or mixtures thereof. The preferred anti-solvent B is water.

Robenacoxib is preferably dissolved in solvent system A at temperatures in the range between 10°C and the refluxing temperature of the solvent system A, more preferably between 25°C and the refluxing temperature. According to another embodiment, dissolution is performed at a temperature of from 20°C to 50°C, preferably 25°C to 40°C.

The term "refluxing temperature of a solvent system" as used herein relates to a temperature in the range of the boiling temperature of the particular solvent to the boiling temperature plus 15°C, i.e. the reflux temperature of ethanol is in the range of from 78°C to 93°C. If a mixture of solvents is used, the "refluxing temperature of a solvent system" as used herein may relate to a temperature in the range of the boiling temperature of the particular solvent mixture to the boiling temperature plus 15°C.

The amounts of robenacoxib and solvent system A used correspond to a solute:solvent weight ratio of less than 1:20, preferably less than 1:10 and more preferred less than 1:7 and most preferred less than 1:6 even more preferred less than 1:5, preferably more than 1:200 and more preferably more than 1:100. In some embodiments, the weight ratio is such that the resulting solution is highly concentrated while ensuring that a clear solution can be obtained. According to such embodiments, for each part by weight of robenacoxib 20 parts by weight or less, preferably 10 parts by weight or less, more preferably 7 parts by weight or less, even more preferably 6 parts by weight or less and most preferably 5 parts by weight or less of solvent are used.

The solvent system A to solvent system B volume ratio can vary from 1:10 to 10: 1, preferably from 5:1 to 1:5, more preferably from 2:1 to 1:2.

In step (iv) the nucleation temperature of the crystallization mixture can be controlled in the following ways:
a) a solution of robenacoxib in a suitable solvent system A, is gradually cooled down to a crystallization temperature of the crystallization mixture and/or solvent B having the same temperature is added to the mixture or vice versa, or
b) to a solution of robenacoxib in a suitable solvent system A, having higher temperature than the crystallization temperature, solvent B having lower temperature than the crystallization temperature is added in controlled manner to maintain the crystallization temperature of the solvent mixture, or
c) to a solution of robenacoxib in a suitable solvent system A, having lower temperature than the crystallization temperature, solvent B having higher temperature than the crystallization temperature is added in controlled manner to maintain the crystallization temperature of the solvent mixture, or
d) a solution of robenacoxib in a suitable solvent system A, having higher temperature than the crystallization temperature, is added into solvent B having lower temperature than the crystallization temperature in controlled manner to maintain the crystallization temperature of the solvent mixture, or
e) a solution of robenacoxib in a suitable solvent system A, having lower temperature than the crystallization temperature, is added into solvent B having higher temperature than the crystallization temperature in controlled manner to maintain the crystallization temperature of the solvent mixture.

In the above embodiments b) to e), it is preferred that the "higher temperature" is in the range of 20°C to 40°C, more preferably 22°C to 30°C, and it is preferred that the "lower temperature" is in the range of 0°C to 10°C, more preferably 2°C to 8°C.

Another aspect of the invention is a process for the preparation of crystallographically essentially pure polymorphic form D2 of robenacoxib according to the present invention which comprises crystallization from the solvent system wherein crystallization temperature is below 50°C or from 40°C to -10°C such as such as 38°C to -9°C, 36°C to -7°C, 33°C to -6°C or 30°C to -5°C, preferably 30°C to 0°C, more preferably in the range from 10°C to 5°C and wherein crystallization at crystallization temperature is induced by cooling the crystallization mixture or evaporating the solvent from the crystallization mixture.

Another aspect of the present invention is the process for the preparation of crystallographically essentially pure polymorphic form D2 of robenacoxib which comprises:
(i) dissolving or suspending robenacoxib in a suitable solvent system at a temperature in the range of from 0°C to refluxing temperature of the crystallization mixture,
(ii) optionally cooling the crystallization mixture to a crystallization temperature below 50°C or optionally evaporating the solvent to initiate crystallization at temperature below 50°C (in this connection and elsewhere in the present disclosure, references to evaporation of the solvent are not particularly limited in terms of the amount of solvent to be evaporated and they may thus mean partial or complete evaporation) or optionally adding one or more seed crystals of form D2 of robenacoxib at a temperature below 50°C
(iii) holding the crystallization mixture for a holding time at crystallization temperature optionally with adding one or more seed crystals of form D2 of robenacoxib and
(iv) separating and drying the crystallized polymorphic form D2 of robenacoxib.

The crystallization temperature in step (ii) and (iii) is preferably below 40°C such as 38°C or less, 36°C or less or 33°C or less, more preferably below 25°C, even more preferably below 15°C, most preferably below 10°C, also it is preferably above -10°C, more preferably above -5°C and even more preferably it is in the range of 7 to 3°C or 0 to -3°C.

In some embodiments, the meaning of the term "crystallization temperature" is the same as defined above for the antisolvent embodiment.

According to the invention, the solvent system is selected from C₁-C₆ alcohols, such as methanol, 1-propanol, 2-propanol, aromatic hydrocarbons, such as toluene, xylene, nitriles, such as acetonitrile. The preferred solvent system comprises or consists of toluene. Most preferably, the solvent system consists of toluene.

The crystallization batch is kept at the crystallization temperature for a holding time for crystal formation for at least 10 minutes, preferably in the range of 10 minutes to 20 hours and more preferred 0.5 to 10 hours. After said holding time at the crystallization temperature, the crystalline particles of robenacoxib are optionally cooled or isolated from the mother liquor using conventional separation techniques, e. g. filtration or centrifugation.

The crystallization process in the context of this aspect of present invention can be induced either by a primary and/or secondary crystallization mechanism.

Primary crystallization in step (ii) can be affected by cooling to and stirring the crystallization mixture at specified crystallization temperatures as long as the precipitation of crystals is initiated or by concentrating the crystallization mixture by evaporation of the solvent.

In case of secondary crystallization, the crystallization batch is seeded by addition of crystals of robenacoxib in pure polymorphic form D2. The crystallization batch is seeded by addition of crystals of robenacoxib at least once during the cooling time or holding time when the temperature of the crystallization batch is within the above defined crystallization temperature range in order to avoid excessive supersaturation with respect to robenacoxib and resulting spontaneous crystallization into non-desired polymorphic forms. The seeding may be repeated in order to ensure constant presence of crystalline robenacoxib during the cooling and/or holding; suitably the crystallization batch can be seeded semi-continuously until crystallization has started. The seeds of robenacoxib can be added in powder form or in form of suspension in any type of liquid.

Stirring of the crystallization batch may be performed either by a magnetic or a mechanical stirrer. In large scale batches preferably, a mechanical stirrer is used. The mechanical stirrer can be selected from impeller, propeller, turbine, paddle or anchor type configuration.

Drying as used herein refers to the removal of solvent residues performed for example by heating and/or vacuum.

Further, the purity of the material obtained by the process of the present invention is more than 98 area% determined by HPLC, preferably more than 99 area% determined by HPLC. The analytical method used for determining the purity of robenacoxib was gradient HPLC method, using XSelect CSH column with stationary phase C18 or a suitable alternative and using UV-detector with detection at wavelength 274 nm. The mobile phase was a combination of water, acetonitrile and formic acid.

Moreover, the process according to this invention can be used in large scale production. It has been confirmed that the process is reliably performed on large scale and in high yield providing robenacoxib in pure polymorphic form D2.

The process according to the present invention yields homogenous particles in the form of readily filterable particles of needle-like morphology or particles of other morphologies, which are suitable to be incorporated directly into the formulation. However, the obtained product may be further subjected to grinding, milling or micronisation if required.

For the milling purposes fluid energy mill, jet mill, ball mill, roller mill or hammer mill are commonly used as milling equipment.

A fluid energy mill, or "micronizer" , is an especially preferred type of mill for its ability to produce particles of small size in a narrow size distribution, i. e., micronized material. As those skilled in the art are aware, micronizers use the kinetic energy of collision between particles suspended in a rapidly moving fluid (typically air or nitrogen) stream to cleave the particles. A jet mill is a preferred fluid energy mill. The suspended particles are injected under pressure into a recirculating particle stream. Smaller particles are carried aloft inside the mill and swept into a vent connected to a particle size classifier such as a cyclone. The feedstock should first be milled to about 30 µm to 100 µm which may be done using a conventional ball, roller, or hammer mill.

The starting material, in particular the material comprising or consisting of robenacoxib and to be adjusted in size, for example by milling, may have an average particle size of about 10 µm to 500 µm.

The material is fed into the micronization system in a controlled feed rate by means of a screw feeder or a vibratory feeder. The jet mill is operated with controlled air or nitrogen pressures. Micronization can also be accomplished with an impact pin mill. The material is fed into the mill system in a controlled feed rate by means of a screw feeder or a vibratory feeder. The mill is operated with controlled speed.

The polymorphic form D2 of the present invention is preferably further characterized by a small particle size determined by average particle size between 1 and 200 µm, preferably 1-100 µm, more preferably 1-50 µm, most preferably 1-30 µm.

Determination and control of particle characteristics, especially the particle size distribution, are essential for product quality control and performance. The term "average particle size" as used herein refers to the volume mean diameter of particles. The diameter and volume mean diameter can be determined by laser light scattering using e.g. a Malvern-Mastersizer Apparatus MS 2000, MS 3000 or equivalent with "wet" dispersion unit. Particle sizes are determined by measuring the angular distribution of laser light scattered by a homogeneous suspension of particles. The particles to be subjected to particle size measurement are first suspended in an appropriate non-polar dispersant and then subjected to size determination in a Malvern Mastersizer instrument. Usually, 100-800 mg of substance is dispersed in 5-10 ml of dispersant. The particle size distribution may also be determined by other method, i.e. analysis of SEM (scanning electron microscope) images. The image analysis process for determining the size distribution of the particles consists of a visual count of all the crystals appearing in an SEM image containing at least 200 particles. The size determined for each particle is that of the largest cross section of said particle concerned.

The process according to the present invention is a robust, reproducible and scalable crystallization process that generates robenacoxib in pure crystalline form D2. Constant polymorphic purity in both the drug substance and in drug product as well as controlling the amorphous content in the drug substance assures product quality and reliability.

Robenacoxib used in the respective processes for the preparation of the polymorphic form D2 may be prepared according to any method known to the skilled person. The preparation of robenacoxib is outlined for example in EP1007505B1, CN102311355, EP1216226, Tetrahedron 2004, Vol 60, Nr 50, Pages 11571 - 11586. Preferably, robenacoxib is used, which has a high purity, preferably a purity of 98% or more, more preferably 99% or more, even more preferably 99.5% or more and most preferably 99.7% or more.

Polymorphic form D2 of robenacoxib prepared in accordance with the process of the present invention can be incorporated into pharmaceutical compositions or formulations as an active ingredient. Of particular advantage is that the present polymorphic form is surprisingly stable when subjected to standard technological processes used in the preparation of pharmaceutical formulations.

The pharmaceutical compositions are preferably solid pharmaceutical compositions in the form of tablets comprising polymorphic form D2 of robenacoxib together with excipients such as microcrystalline cellulose, powdered cellulose, crospovidone, povidone, magnesium stearate and optionally flavor such as artificial beef flavor and yeast powder.

In an alternative embodiment, the pharmaceutical compositions are solid pharmaceutical compositions comprising polymorphic form D2 of robenacoxib which are intended to be dissolved prior to use to prepare pharmaceutical compositions in the form of a solution, preferably a solution for injection comprising robenacoxib, Macrogol 400, ethanol, anhydrous, Poloxamer 188, citric acid monohydrate, sodium metabisulphite, sodium hydroxide and water for injections.

The following examples illustrate the invention and are not intended to restrict the scope of the invention in any way.

### Examples

The compounds according to the present invention were characterized with regard to their **X-ray powder diffraction patterns** (obtained by a PANalytical X'Pert PRO diffractometer using CuKα radiation of 1.541874 Ⓐ).

In the following examples, robenacoxib having a purity of 99.8% was used as a starting material. The robenacoxib used as a starting material was a mixture of polymorphic form D1 and polymorphic form D2.

**SEM images** were acquired under high vacuum on a scanning electron microscope CarlZeiss Ultra Plus The accelerating voltage was 1-2 kV and the working distance was 2.1-5.3 mm.

**The differential scanning calorimetry (DSC)** was carried out using DSC 1 Mettler Toledo scanning calorimeter. Samples of approx. 3 mg were scanned between 20°C and 270°C at a heating rate of 10°C/min under nitrogen atmosphere (40ml/min) in aluminium pans, covered with pierced lid.

**Termogravimetric (TG)** analysis was performed on TGA/DSC1 Mettler Toledo analyzer. Samples of approx. 7 - 15 mg were canned between 20°C and 220°C at a heating rate of 10°C / min under nitrogen atmosphere in aluminum crucibles with perforated lid.

**Dynamic vapor sorption** analysis was performed on dynamic vapor sorption analyzer ProUmid GmbH, SPS 1µ. Up to 100 mg of samples were exposed to relative humidity from 0 % to 90 % RH and from 90 % RH back to 0 % in two cycles at 25 °C in steps of 10% RH. Minimum time of 360 minutes at each step was chosen.

**Hot stage microscopy** was performed using a Mettler FP82MT stage with FP90 controller mounted on a Nicon Eclipse E600 microscope. Stage was precisely calibrated at 3 temperature points. Samples were placed on a cover glass, and a second cover glass was placed on top of the sample. Samples were heated to 170°C until reaching melting point using temperature ramp of 10 °C/min. As stage was heated, each sample was visually observed.

### Reference Example 1

Ethyl acetate and hexane in volume ratio 40:60 (1 mL) were charged into a glass reactor at room temperature. Robenacoxib (0.11 g) was added into the reactor, and the mixture was heated to 70 °C until robenacoxib was dissolved. The solvent was evaporated. The isolated product was dried in a vacuum dryer under reduced pressure (500 mbar) and a temperature of 25 °C overnight. 50 mg of material was obtained. The obtained product was polymorphic form D1.

### Reference Example 2

317.5 mg of robenacoxib were charged into a glass reactor, and 0.5 ml of cyclohexane were added to obtain a suspension. The suspension was heated to 80°C and additional 2.5 ml cyclohexane was added. The suspension was gradually cooled to room temperature in 3 hours. The obtained product was isolated with suction filtration using a Büchner funnel. The isolated product was dried in a vacuum dryer under reduced pressure (500 mbar) and a temperature of 25 °C overnight. 110 mg of material was obtained. The obtained product was polymorphic form D1.

### Reference Example 3

0.80 g of robenacoxib were charged into a glass reactor. 9 ml of ethanol and 0.5 ml of water were added and the mixture was heated to reflux (78 °C). 0.95 g (0,7 ml) 30 % w/w NaOH were dropwise slowly added and heated at reflux temperature for an hour. The solution was cooled to 40 °C and 0.5 M HCl was slowly added until pH 3-4 was reached. The obtained suspension was cooled to 20 °C, and isolated with suction filtration using a Büchner funnel. The isolated product was dried in a vacuum dryer under reduced pressure (500 mbar) and a temperature of 25 °C overnight. 740 mg of material as obtained. The obtained product was polymorphic form D1.

### Process for preparing polymorphic form D2 by solvent/anti-solvent system

### Example 1

0.25 g of robenacoxib were charged into a glass reactor and 1.75 ml of water were added. Separately, a solution of 0.25 g of robenacoxib and 1.75 ml of acetone was prepared. The latter was added to the suspension in the glass reactor, heated to 40°C and stirred for 8 h. After that the suspension was cooled to 25°C and stirred for further 5 h. The product was isolated with suction filtration using a Büchner funnel. The isolated product was dried in a vacuum dryer under reduced pressure (500 mbar) and a temperature of 25 °C overnight. 440 mg of material was obtained. The obtained product was polymorphic form D2.

### Example 2

3 ml of water were charged into a glass reactor. Separately, a solution of 0.5 g of robenacoxib in 2 ml of acetone was prepared. The solution was added to the water in the glass reactor at room temperature. The obtained suspension was then heated to 40° C and stirred for 8 h at said temperature, followed by cooling of the suspension to room temperature and stirring for further 5 h. The obtained product was isolated with suction filtration using a Büchner funnel. The isolated product was dried in a vacuum dryer under reduced pressure (500 mbar) and a temperature of 25 °C overnight.

340 mg of material was obtained. The obtained product was polymorphic form D2, average particle size is about 380 µm as determined by scanning electron microscope, SEM images of the product is shown in Figure 8.

### Example 3

A solution of 0.25 g of robenacoxib in 2 ml of acetone was prepared. The solution was then slowly added to a reactor comprising 3 ml of water cooled to 5°C, maintaining the temperature of the crystallization mixture at 5°C. The suspension thus prepared was then stirred for 3 hours and then the product was isolated using Büchner funnel. 140 mg of material was obtained. The obtained product was polymorphic form D2.

### Example 4

A solution of 1.5 g of robenacoxib in 6 ml of acetone was prepared. The solution was then dropwise slowly added to the reactor comprising 9 ml of water cooled to 5°C, maintaining the temperature of the crystallization mixture at 5°C. The suspension thus prepared was then stirred at this temperature for 4 hours and then the product was isolated using a Büchner funnel. The isolated product was dried in a vacuum dryer under reduced pressure (500 mbar) and a temperature of 25 °C overnight.

1.1 g of material was obtained. The obtained product was polymorphic form D2, average particle size is 5-10 µm as determined by scanning electron microscope.

### Example 5

A solution of 0.54 g of robenacoxib in 2 ml of acetone was prepared at room temperature. The solution was dropwise slowly added into 3 ml of water heated to 30°C. The suspension was then cooled slowly in 4 hours to room temperature and the product was isolated using a Büchner funnel. 400 mg of material was obtained. The obtained product was polymorphic form D2.

### Example 6

A solution of 0.52 g of robenacoxib in 2 ml of acetone was prepared at room temperature. The solution was dropwise slowly added into 3 ml water heated to 40°C. The suspension was then cooled slowly in 4 hours to room temperature and isolated using a Büchner funnel.410 mg of material was obtained. The obtained product was a mixture polymorphic form D2 and D1.

### Example 7

A solution of 0.52 g of robenacoxib in 2 ml of acetone was prepared at room temperature. The solution was dropwise slowly added into 3 ml of water heated to 50°C. The suspension was then cooled slowly in 4 hours to room temperature and the product was isolated using a Büchner funnel. 420 mg of material was obtained. The obtained product was a mixture of polymorphic form D2 and D1.

### Example 8

A solution of 10.04 g of robenacoxib in 40 ml of acetone was prepared at room temperature. The solution was added to 60 ml of water cooled to 5°C. The suspension was stirred at said temperature for 4 hours. The product was isolated using a Büchner funnel and dried in a vacuum dryer under reduced pressure (500 mbar) and a temperature of 25°C overnight. 9,3 g of material was obtained. The obtained product was polymorphic form D2, average particle size is 5-10 µm as determined by scanning electron microscope, SEM image of the product is shown in Figure 9.

### Example 9

0.50 g of robenacoxib were charged into a 25 mL round bottom flask and 2.27 mL of methyl isobutyl ketone (MIBK) were added to obtain a solution at room temperature. The solution was then cooled down to 5°C. Then 4.5 mL of dichloromethane were added to the solution slowly in drops to obtain a suspension. The obtained suspension was filtered and the product was placed to a vacuum dryer at 22°C and 500 mbar. 0.07 g of product was obtained. The obtained product was polymorphic form D2.

### Example 10

1.01 g of robenacoxib were charged into a 5 mL round bottom flask and 4 mL of acetone were added to obtain a solution at room temperature. Meanwhile 6 mL of water were charged to a 25 mL round bottom flask and cooled down to 5°C. The robenacoxib/acetone solution was then slowly added to the cooled water dropwise and stirred for 4h at temperature of 5°C. The suspension was then filtered and washed with 5mL of cooled water. The product was placed to a vacuum dryer at 22°C and 500 mbar. 0.88 g of product was obtained. The obtained product was polymorphic form D2.

### Example 11

1.01 g of robenacoxib were charged into a 25 mL round bottom flask and 4 mL of acetone were added to obtain a solution at room temperature. Meanwhile 6 mL of water were charged to a 10 mL round bottom flask and cooled down to 5°C. The cooled water was then slowly added dropwise to the solution and stirred for 4h at temperature of 5°C. The suspension was then filtered and washed with 5mL of cooled water. The product was placed to a vacuum dryer at 22°C and 500 mbar. 0.84 g of product was obtained. The obtained product was polymorphic form D2.

### Example 12

12.03 g of robenacoxib were charged into a 100 mL round bottom flask and 48 mL of acetone were added to obtain a solution at room temperature. When robenacoxib was completely dissolved the solution was charged to a reactor. Then 72 mL of water cooled to 5°C were slowly added to the solution. After the end of the addition the suspension was cooled to 0°C and stirred overnight. The suspension was then filtered and washed with 10mL of cooled water. The product was placed to a vacuum dryer at 22°C and 500 mbar. 11.69 g of product was obtained. The obtained product was polymorphic form D2, average particle size is about 45 µm as determined by scanning electron microscope.

### Process for preparing polymorphic form D2 by a method different than solvent/anti-solvent system

### Example 13

0.5 g of robenacoxib in polymorphic form D1 and 5 ml of toluene were added into a glass reactor. The suspension was then heated to 40°C and stirred for 8 h at said temperature, followed by cooling of the suspension to room temperature and stirred for further 5 h. The obtained product was isolated with suction filtration using a Büchner funnel. The isolated product was dried in a vacuum dryer under reduced pressure (500 mbar) and a temperature of 25°C overnight. 370 mg of material was obtained. The obtained product was polymorphic form D2.

### Example 14

0.72 g of robenacoxib were dissolved in 5 ml of methanol in a glass reactor. The solution was heated to 40°C and then slowly cooled to -10°C. The material started to crystallize between 0°C and -10°C. The obtained product was isolated with suction filtration using a Büchner funnel. The isolated product was dried in a vacuum dryer under reduced pressure (500 mbar) and a temperature of 25°C overnight. 110 mg of material was obtained. The obtained product was polymorphic form D2.

### Example 15

In a glass reactor, 0.51 g of robenacoxib in polymorphic form D1 were suspended in 5 ml of toluene at room temperature and rapidly cooled to 5°C. The suspension was stirred at said temperature overnight. The obtained product was isolated with suction filtration using a Büchner funnel. The isolated product was dried in a vacuum dryer under reduced pressure (500 mbar) and a temperature of 25°C overnight. 460 mg of material was obtained. The obtained product was polymorphic form D2.

### Example 16

In a glass reactor, 0.58 g of robenacoxib were dissolved in 5 ml of 2-propanol at room temperature. The solution was then rapidly cooled to 5°C and stirred at this temperature overnight. The obtained product was isolated with suction filtration using a Büchner funnel. The isolated product was dried in a vacuum dryer under reduced pressure (500 mbar) and a temperature of 25°C overnight. 150 mg of material was obtained. The obtained product was polymorphic form D2.

### Example 17

0.50 g of robenacoxib were charged into a 25 mL round bottom flask and 10 mL of acetonitrile were added to obtain a solution at room temperature. The solution was then cooled down to 5°C to obtain a suspension and stirred for 1h. The obtained suspension was filtered and the product was placed to a vacuum dryer at 22°C and 500 mbar. 0.07 g of product was obtained. The obtained product was polymorphic form D2.

### Example 18

0.53 g of robenacoxib were charged into a 25 mL round bottom flask and 1.85 mL of acetone were added to obtain a solution at room temperature. The solution was then cooled down to 5°C. Then 3.7 mL of toluene were added to the solution slowly in drops. Since the solution remained clear even after the 6 times amount of the anti-solvent (22.2 mL in total), the solvent was slowly (partially) evaporated with nitrogen. The product was placed to a vacuum dryer at 22°C and 500 mbar. 0.24 g of product was obtained. The obtained product was polymorphic form D2.

### Example 19

10.03 g of robenacoxib and 100 mL of toluene were charged into a 100 mL reactor to obtain a suspension at room temperature. The suspension was then cooled to 5°C and stirred overnight. The suspension was then filtered and the product was placed to a vacuum dryer at 22°C and 500 mbar. 9.16 g of product was obtained. The obtained product was polymorphic form D2, average particle size is about 109 µm as determined by scanning electron microscope.

## Claims

1. A process for the preparation of crystallographically essentially pure polymorphic form D2 of robenacoxib which comprises:
(i) dissolving robenacoxib in a suitable solvent system A at temperature in the range of from 0°C to refluxing temperature of said organic solvent system;
(ii) optionally filtering the obtained crystallization mixture;
(iii) optionally cooling the crystallization mixture to a temperature in the range of from 0°C to 50°C or from 40°C to -10°C, preferably 30°C to 0°C;
(iv) then
a. addition of an anti-solvent B to the crystallization mixture to initiate precipitation at crystallization temperature in the range from 50°C to 0°C or from 40°C to -10°C, preferably 30°C to 0°C, or
b. addition of the crystallization mixture to an anti-solvent B, to initiate precipitation at crystallization temperature in the range from 50°C to 0°C or from 40°C to -10°C, preferably 30°C to 0°C;
(v) optionally cooling the obtained suspension;
(vi) separating and drying the crystallized polymorphic form D2 of robenacoxib;
wherein the solvent system A is selected from the group consisting of:
• ketones, such as acetone, methyl ethyl ketone and methyl isobutyl ketone,
• and mixtures thereof;
wherein the anti-solvent B is selected from the group consisting of:
• water,
• halogenated hydrocarbons, such as dichloromethane,
• and mixtures thereof;
wherein the term crystallographically essentially pure means that the polymorphic form comprises less than 30 wt.% of other polymorphic forms, amorphous form or solvates, wherein polymorphic purity is determined by means of X-ray powder diffraction, and wherein polymorphic form D2 of robenacoxib is **characterized by** an X-ray diffraction pattern obtained using CuKα radiation and comprising signals with peak positions at the following 2θ values: 6.0°, 10.1°, 17.0°, 22.5° and 29.1°.

2. The process according to claim 1 wherein solvent system A is acetone and anti-solvent B is water.

3. The process according to claim 1 or 2 wherein crystallization temperature is in the range from 30°C to 0°C.

4. A process for the preparation of crystallographically essentially pure polymorphic form D2 of robenacoxib which comprises:
(i) dissolving or suspending robenacoxib in a suitable solvent system at a temperature in the range from 0°C to refluxing temperature of the crystallization mixture;
(ii) optionally cooling the crystallization mixture to a crystallization temperature below 50°C or optionally evaporating the solvent to initiate crystallization at temperature below 50°C or optionally adding one or more seed crystals of form D2 of robenacoxib at a temperature below 50°C;
(iii) holding the crystallization mixture for a holding time at crystallization temperature optionally with adding one or more seed crystals of form D2 of robenacoxib; and
(iv) separating and drying the crystallized polymorphic form D2 of robenacoxib;
wherein the solvent system is selected from the group consisting of:
• C₁-C₆ alcohols, such as methanol, 1-propanol and 2-propanol,
• aromatic hydrocarbons, such as toluene and xylene, and
• nitriles, such as acetonitrile;
wherein the term crystallographically essentially pure means that the polymorphic form comprises less than 30 wt.% of other polymorphic forms, amorphous form or solvates, wherein polymorphic purity is determined by means of X-ray powder diffraction, and wherein polymorphic form D2 of robenacoxib is **characterized by** an X-ray diffraction pattern obtained using CuKα radiation and comprising signals with peak positions at the following 2θ values: 6.0°, 10.1°, 17.0°, 22.5° and 29.1°.

5. The process according to claim 4, wherein the solvent system is toluene.

6. The process according to any of claims 1 to 5, which further contains the step of combining the crystallized polymorphic form D2 of robenacoxib with one or more pharmaceutically acceptable excipients to obtain a pharmaceutical composition.

## Patentansprüche

1. Ein Verfahren zur Herstellung der kristallographisch im Wesentlichen reinen polymorphen Form D2 von Robenacoxib, welches umfasst:
(i) Auflösen von Robenacoxib in einem geeigneten Lösungsmittelsystem A bei einer Temperatur im Bereich von 0 °C bis zur Rückflusstemperatur des genannten organischen Lösungsmittelsystems;
(ii) gegebenenfalls Filtrieren des erhaltenen Kristallisationsgemisches;
(iii) gegebenenfalls Abkühlen des Kristallisationsgemisches auf eine Temperatur im Bereich von 0 °C bis 50 °C oder von 40 °C bis -10 °C, vorzugsweise von 30 °C bis 0 °C;
(iv) anschließend
a. Zugabe eines Antisolvens B zum Kristallisationsgemisch, um die Ausfällung bei einer Kristallisationstemperatur im Bereich von 50 °C bis 0 °C oder von 40 °C bis -10 °C, vorzugsweise von 30°C bis 0 °C, einzuleiten, oder
b. Zugabe des Kristallisationsgemisches zu einem Antisolvent B, um die Ausfällung bei einer Kristallisationstemperatur im Bereich von 50 °C bis 0 °C oder von 40 °C bis -10 °C, vorzugsweise von 30 °C bis 0 °C, einzuleiten;
(v) gegebenenfalls Abkühlen der erhaltenen Suspension;
(vi) Abtrennung und Trocknung der kristallisierten polymorphen Form D2 von Robenacoxib;
wobei das Lösungsmittelsystem A ausgewählt ist aus der Gruppe bestehend aus:
• Ketonen, wie Aceton, Methylethylketon und Methylisobutylketon,
• und Mischungen davon;
wobei das Antisolvent B aus der Gruppe ausgewählt ist, bestehend aus:
• Wasser,
• halogenierten Kohlenwasserstoffen, wie beispielsweise Dichlormethan,
• und Mischungen davon;
wobei der Begriff "kristallographisch im Wesentlichen rein" bedeutet, dass die polymorphe Form weniger als 30 Gew.-% anderer polymorpher Formen, amorpher Formen oder Solvate enthält, wobei die polymorphe Reinheit mittels Röntgenpulverdiffraktometrie bestimmt wird und wobei die polymorphe Form D2 von Robenacoxib durch ein Röntgendiffraktionsmuster charakterisiert ist, das unter Verwendung von CuKα-Strahlung erhalten wurde und Signale mit Peakpositionen bei den folgenden 2θ-Werten umfasst: 6,0°, 10,1°, 17,0°, 22,5° und 29,1°

2. Das Verfahren nach Anspruch 1, wobei das Lösungsmittelsystem A Aceton und das Antisolvent B Wasser ist.

3. Das Verfahren nach Anspruch 1 oder 2, wobei die Kristallisationstemperatur im Bereich von 30 °C bis 0 °C liegt.

4. Ein Verfahren zur Herstellung der kristallographisch im Wesentlichen reinen polymorphen Form D2 von Robenacoxib, welches umfasst:
(i) Auflösen oder Suspendieren von Robenacoxib in einem geeigneten Lösungsmittelsystem bei einer Temperatur im Bereich von 0 °C bis zur Rückflusstemperatur des Kristallisationsgemisches;
(ii) gegebenenfalls Abkühlen des Kristallisationsgemisches auf eine Kristallisationstemperatur unter 50 °C oder gegebenenfalls Verdampfen des Lösungsmittels, um die Kristallisation bei einer Temperatur unter 50 °C einzuleiten, oder gegebenenfalls Hinzufügen eines oder mehrerer Impfkristalle der Form D2 von Robenacoxib bei einer Temperatur unter 50 °C;
(iii) Halten des Kristallisationsgemisches für eine Haltezeit bei der Kristallisationstemperatur, gegebenenfalls unter Zugabe eines oder mehrerer Impfkristalle der Form D2 von Robenacoxib; und
(iv) Abtrennung und Trocknung der kristallisierten polymorphen Form D2 von Robenacoxib;
wobei das Lösungsmittelsystem aus der Gruppe ausgewählt ist, bestehend aus:
• C₁-C₆-Alkoholen, wie Methanol, 1-Propanol und 2-Propanol,
• aromatischen Kohlenwasserstoffen, wie Toluol und Xylol, und
• Nitrilen, wie Acetonitril;
wobei der Begriff "kristallographisch im Wesentlichen rein" bedeutet, dass die polymorphe Form weniger als 30 Gew.-% anderer polymorpher Formen, amorpher Formen oder Solvate enthält, wobei die polymorphe Reinheit mittels Röntgenpulverdiffraktometrie bestimmt wird und wobei die polymorphe Form D2 von Robenacoxib durch ein Röntgendiffraktionsmuster charakterisiert ist, das unter Verwendung von CuKα-Strahlung erhalten wurde und Signale mit Peakpositionen bei den folgenden 2θ-Werten umfasst: 6,0°, 10,1°, 17,0°, 22,5° und 29,1°.

5. Das Verfahren nach Anspruch 4, wobei das Lösungsmittelsystem Toluol ist.

6. Das Verfahren nach einem der Ansprüche 1 bis 5, das ferner den Schritt umfasst, die kristallisierte polymorphe Form D2 von Robenacoxib mit einem oder mehreren pharmazeutisch verträglichen Hilfsstoffen zu kombinieren, um eine pharmazeutische Zusammensetzung zu erhalten.

## Revendications

1. Procédé de préparation de la forme polymorphe D2 du robenacoxib, essentiellement pure sur le plan cristallographique, qui comprend:
(i) la dissolution du robenacoxib dans un système de solvants A approprié, à une température comprise entre 0 °C et la température de reflux dudit système de solvants organiques;
(ii) éventuellement, de filtrer le mélange de cristallisation obtenu;
(iii) éventuellement, le refroidissement du mélange de cristallisation à une température comprise entre 0 °C et 50 °C ou entre 40 °C et -10 °C, de préférence entre 30 °C et 0 °C;
(iv) puis
a. ajouter un antisolvant B au mélange de cristallisation pour déclencher la précipitation à une température de cristallisation comprise entre 50 °C et 0 °C ou entre 40°C et -10 °C, de préférence entre 30 °C et 0 °C, ou
b. l'ajout du mélange de cristallisation à un antisolvant B, pour déclencher la précipitation à la température de cristallisation comprise entre 50 °C et 0 °C ou entre 40 °C et -10 °C, de préférence entre 30 °C et 0 °C;
(v) éventuellement, le refroidissement de la suspension obtenue;
(vi) séparation et séchage de la forme polymorphe D2 cristallisée du robenacoxib;
le système de solvants A étant choisi parmi le groupe constitué de:
• des cétones, telles que l'acétone, la méthyléthylcétone et la méthylisobutylcétone,
• et leurs mélanges;
dans lequel l'antisolvant B est choisi parmi le groupe constitué de :
• l'eau,
• les hydrocarbures halogénés, tels que le dichlorométhane,
• et leurs mélanges;
le terme «essentiellement pur sur le plan cristallographique» signifiant que la forme polymorphe comprend moins de 30 % en poids d'autres formes polymorphes, de forme amorphe ou de solvates, la pureté polymorphe étant déterminée par diffraction des rayons X sur poudre, et la forme polymorphe D2 du robenacoxib étant **caractérisée par** un diagramme de diffraction des rayons X obtenu à l'aide d'un rayonnement CuKα et comprenant des signaux dont les pics se situent aux valeurs 2θ suivantes: 6,0°, 10,1°, 17,0°, 22,5° et 29,1°

2. Procédé selon la revendication 1, dans lequel le système de solvants A est constitué d'acétone et l'antisolvant B est de l'eau.

3. Procédé selon la revendication 1 ou 2, dans lequel la température de cristallisation est comprise dans la plage allant de 30 °C à 0 °C.

4. Procédé de préparation de la forme polymorphe D2 du robenacoxib, essentiellement pure sur le plan cristallographique, qui comprend :
(i) la dissolution ou la mise en suspension du robenacoxib dans un système de solvants approprié à une température comprise entre 0 °C et la température de reflux du mélange de cristallisation;
(ii) éventuellement, le refroidissement du mélange de cristallisation à une température de cristallisation inférieure à 50 °C, ou éventuellement l'évaporation du solvant pour déclencher la cristallisation à une température inférieure à 50 °C, ou éventuellement l'ajout d'un ou plusieurs cristaux d'ensemencement de la forme D2 du robenacoxib à une température inférieure à 50 °C;
(iii) maintenir le mélange de cristallisation pendant un certain temps à la température de cristallisation, en ajoutant éventuellement un ou plusieurs cristaux d'ensemencement de la forme D2 du robenacoxib; et
(iv) séparer et sécher la forme polymorphe D2 cristallisée du robenacoxib;
le système de solvants étant choisi parmi le groupe constitué de:
• des alcools en C₁à C₆, tels que le méthanol, le 1-propanol et le 2-propanol,
• des hydrocarbures aromatiques, tels que le toluène et le xylène, et
• des nitriles, tels que l'acétonitrile;
où l'expression «essentiellement pur du point de vue cristallographique» signifie que la forme polymorphe comprend moins de 30 % en poids d'autres formes polymorphes, de forme amorphe ou de solvates, la pureté polymorphe étant déterminée par diffraction des rayons X sur poudre, et où la forme polymorphe D2 du robenacoxib est **caractérisée par** un diagramme de diffraction des rayons X obtenu à l'aide d'un rayonnement CuKα et comprenant des signaux dont les pics se situent aux valeurs 2θ suivantes: 6,0°, 10,1°, 17,0°, 22,5° et 29,1°.

5. Procédé selon la revendication 4, dans lequel le système de solvants est le toluène.

6. Procédé selon l'une quelconque des revendications 1 à 5, qui comprend en outre l'étape consistant à associer la forme polymorphe D2 cristallisée du robenacoxib à un ou plusieurs excipients pharmaceutiquement acceptables afin d'obtenir une composition pharmaceutique.
